(19) Europäisches Patentamt
European Patent Office
Office européen des brevets

(11) Publication number: **0 395 302 B1**

(12) # EUROPEAN PATENT SPECIFICATION

(45) Date of publication of patent specification: **04.01.95** (51) Int. Cl.[6]: **C07D 281/10**

(21) Application number: **90304200.0**

(22) Date of filing: **10.04.90**

(54) **Process for preparing 1,5-benzothiazepine derivatives.**

(30) Priority: **28.04.89 JP 109794/89**

(43) Date of publication of application:
**31.10.90 Bulletin 90/44**

(45) Publication of the grant of the patent:
**04.01.95 Bulletin 95/01**

(84) Designated Contracting States:
**AT BE CH DE DK ES GB GR IT LI LU NL SE**

(56) References cited:
**EP-A- 0 343 474**
**EP-A- 0 378 455**

(73) Proprietor: **TANABE SEIYAKU CO., LTD.**
**2-10, Dosho-machi 3-chome**
**Chuo-ku**
**Osaka (JP)**

(72) Inventor: **Nishimoto, Shigeru**
**B-110, 16 Aogein 1-chome**
**Minoo-shi,**
**Osaka-Fu (JP)**
Inventor: **Nakao, Akio**
**15-4, Abeno-motomachi,**
**Abeno-ku**
**Osaka-shi,**
**Osaka (JP)**
Inventor: **Ikeda, Yasuji**
**501, 7-8 Higashino-Kitsuneyabu-cho,**
**Yamashina-ku**
**Kyoto-shi,**
**Kyoto-fu (JP)**
Inventor: **Nate, Hiroyuki**
**304, 9-3 Kosaka-Honmachi 2-chome**
**Higashiosaka-shi,**
**Osaka-fu (JP)**

(74) Representative: **Hardisty, David Robert et al**
**BOULT, WADE & TENNANT**
**27 Furnival Street**
**London EC4A IPO (GB)**

**EP 0 395 302 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel process for preparing 1,5-benzothiazepine derivatives represented by the formula:

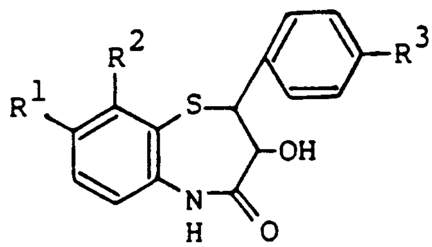

(I)

wherein one of $R^1$ and $R^2$ is a $C_1$-$C_4$ alkyl group or halogen atom, and the other is hydrogen, $R^3$ is a $C_1$-$C_4$ alkyl group or a $C_1$-$C_4$ alkoxy group.

The above 1,5-benzothiazepine derivatives (I) are useful as an intermediate for the synthesis of, for example, the corresponding 3-acetoxy-5-[2-(dimethylamino)ethyl]-1,5-benzothiazepine derivatives having excellent hypotensive activity.

Heretofore, as a process for preparing 1,5-benzothiazepine derivatives (I), the method in which a propionic acid derivatives represented by the formula:

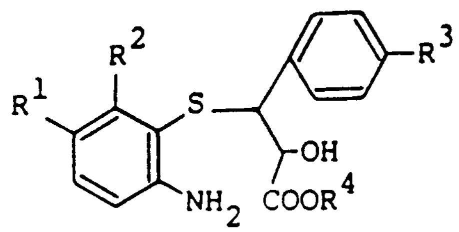

(II)

wherein $R^4$ represents hydrogen atom or an ester residue, and $R^1$, $R^2$ and $R^3$ have the same meanings as defined above,
is heated in a solvent (for example, xylene) to effect intramolecular ring closing has been known (U.S. Patents No. 4,567,175 and No. 4,590,188). However, this method involves the problem of requiring a long period of time for the intramolecular ring closing reaction.

EP-A-0 343474 and EP-A-378455 are comprised in the state of the art in accordance with Article 54(3) EPC.

EP-A- 0 343 474 discloses a process for preparing an acid of the general formula

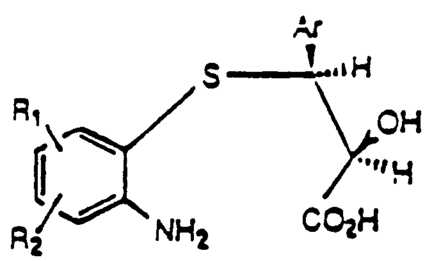

wherein $R_1$ and $R_2$ are each, independently, hydrogen, alkyl of 1 to 4 carbon atoms, alkoxy of 1 to 4 carbon atoms, halogen, trifluoromethyl or nitro or $R_1$ and $R_2$ taken together with the benzene ring to which they are attached, are naphthalene and Ar is phenyl which unsubstituted or substituted by one to three substituents selected from the group consisting of alkyl of 1 to 4 carbon atoms, alkoxy of 1 to 4 carbon atoms and halogen,
which comprises hydrolyzing a compound of the general formula

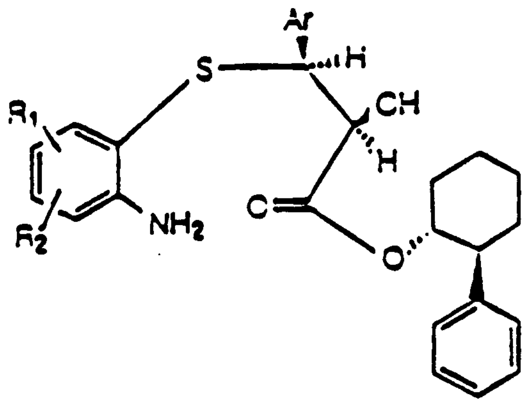

wherein $R_1$ and $R_2$ and Ar are as described above, by reacting same with a base in a polar, protic solvent. It may be noted that in the paragraph of this prior specification at lines 21 to 37, page 4, there is disclosed the use for cyclization of an organic acid catalyst such as p-toluenesulfonic acid monohydrate.

Furthermore, EP-A-0 378 455 discloses a process for the preparation of a compound of the general formula

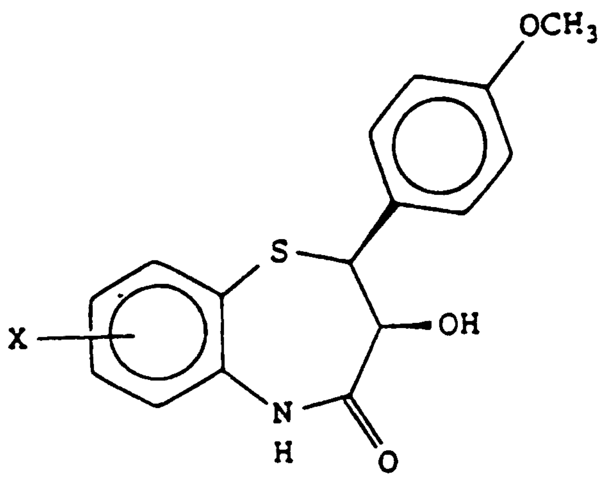

(Ia)

in which X represents one atom of hydrogen or chlorine, comprising firstly causing a 2-aminothiophenol of the general formula

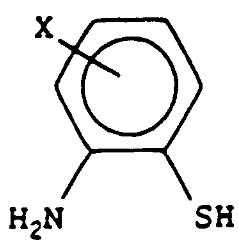

in which X is defined above, to react with (-)-(2R,3S)-2,3-epoxy-3-(4-methoxylproprionate of formula

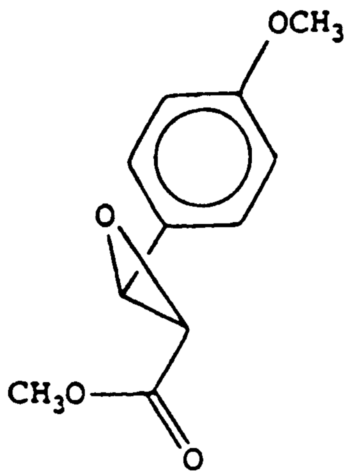

(IIIa)

then cyclising the intermediate compound thus obtained, of general formula

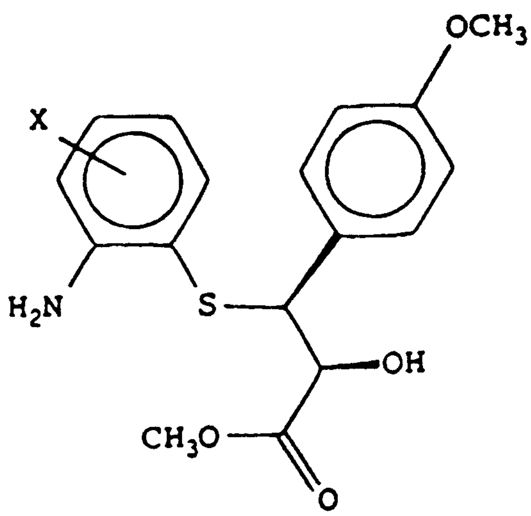

(IVa)

in the presence of acid, which process is characterised in that the two reaction steps are carried out, without isolation the intermediate compound, using a solvent chosen from among the chlorinated solvents having a boiling point above 70 ° C.

The present inventors have studied various research and as a result, they have found that, when the intramolecular ring closing reaction of the compound (II) is carried out in the presence of a specific sulfonic acid compound, the compound (I) can be prepared by the reaction within a short time with good yield. The present invention has been established based on such findings.

According to the invention there is provided a process for preparing 1,5-benzothiazepine derivatives represented by the formula:

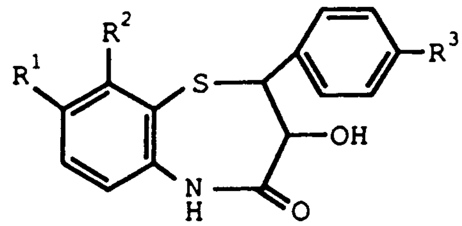

(I)

wherein one of $R^1$ and $R^2$ is a $C_1$ - $C_4$ alkyl group or halogen atom, and the other is hydrogen atom, $R^3$ is a $C_1$ - $C_4$ alkyl group or a $C_1$ - $C_4$ alkoxy group,
which comprises subjecting a propionic acid compound represented by the formula (II):

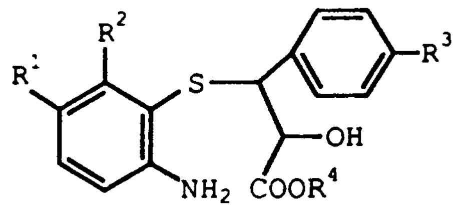

(II)

wherein $R^1$, $R^2$ and $R^3$ have the same meanings as defined above, and $R^4$ represents an ester residue, provided that (1R,2S)-2-phenylcyclohexyl group is disclaimed from said ester residue,
to intromolecular ring closing reaction in the presence of sulfonic acid compound represented by the formula (III)

$R^5SO_3H$ (III)

wherein $R^5$ represents methyl group, ethyl group, propyl group, butyl group or phenyl group which may be substituted by at least one selected from methyl group, ethyl group, proply group and butyl group, with the proviso that the specific combination of one of $R^1$ and $R^2$ is chlorine atom and the other is hydrogen atom, $R^3$ is methoxy group and $R^4$ is methyl group, and the solvent being chlorinated with a boiling point over 70 °C and $R^5$ is methyl group or p-tolyl group is disclaimed.

Examples of the sulfonic acid compound (III) to be used in the intramolecular ring closing reaction of the present invention include, for example, the compounds wherein $R^5$ in the formula (III) is an alkyl group having 1 to 4 carbon atoms such as methyl group, ethyl group, propyl group or butyl group or a phenyl group which may be substituted by at least one of these alkyl groups, and particularly, methanesulfonic acid or p-toluenesulfonic acid is preferably used. An amount of the said sulfonic acid is not particularly limited but generally it is preferably used at an amount of 0.5 to 10 w/w %, more preferably about 1 to about 6 w/w % based on the compound (II).

The present intramolecular ring closing reaction is preferably practiced in an appropriate solvent under reflux. As the solvent, high boiling point solvents such as xylene, toluene and dichlorobenzene are preferably used, and xylene is particularly preferably used. A reaction time may be extremely short as compared with the case where no sulfonic acid compound is present in the reaction system, and for example, when xylene is used as a solvent, the reaction can be terminated at about 30 minutes to about 4 hours.

The desired compound (I) formed can be isolated as a pure product containing no sulfonic acid compound (III) by simple and easy operations as, for example, cooling the reaction mixture, collecting precipitated crystals by filtration, and washing with a suitable solvent (e.g. ethanol, aqueous ethanol, etc.).

The thus obtained compound (I) can be converted to the corresponding 3-acetoxy-5-(β-dimethylaminoethyl)-1,5-benzothiazepine derivatives represented by the formula:

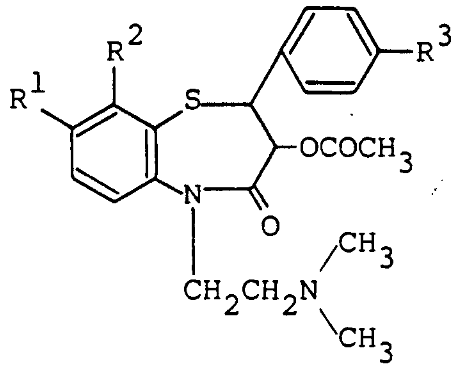

(VI)

wherein $R^1$, $R^2$ and $R^3$ have the same meanings as defined above, or a pharmaceutically acceptable salt thereof in a known method, for example, in a method described in U.S. Patents No. 4,567,175 and No. 4,590,188, the contents of which are hereby incorporated herein by reference.

According to the process of the present invention as mentioned above, the intramolecular ring closing reaction can be terminated within a short period of time and the desired compound can be obtained in high yield and high purity. Therefore, the process of the present invention is extremely excellent from the industrial view point as compared with the conventional process in which the reaction is performed in the absence of the sulfonic acid compound.

The starting compound (II) can be prepared according to the method as disclosed in Japanese Provisional Patent Publication No. 225174/1984 or No. 202871/1985, but an optically active compound (II) wherein $R^4$ is an ester residue, i.e. an optically active threo-propionic acid ester derivative represented by the formula:

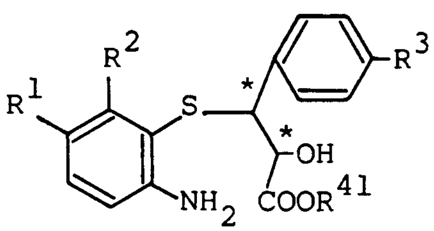

(II - a)

wherein $R^{41}$ represents an ester residue, * represents an asymmetric carbon atom, and $R^1$, $R^2$ and $R^3$ have the same meanings as defined above, can be prepared by reacting a thiophenol compound represented by the formula:

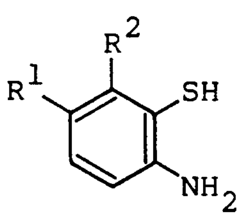

(IV)

wherein symbols have the same meanings as defined above, with an optically active trans-glycidic acid ester represented by the formula:

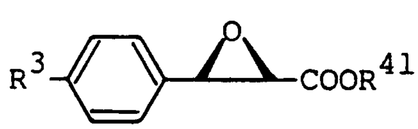

(V)

wherein symbols have the same meanings as defined above.

The above reaction is preferably practiced in an appropriate solvent (e.g. xylene, toluene, etc.) under heating. The compound (II - a) may be isolated, but without isolation, the reaction mixture can be applied to the subsequent intramolecular ring closure reaction. In this case, the optically active desired compound (I) can be obtained from the compound (IV) in the same reaction vessel within a short period of time in good yield. Therefore, the process is industrially extremely advantageous.

Throughout the specification and claims, the term "lower alkyl" and "lower alkoxy" should be interpreted as referring to alkyl having one to four carbon atoms and alkoxy having one to four carbon atoms, respectively.

Example 1

(1) In 400 ml of toluene are dissolved 24.0 g of 2-amino-5-chlorothiophenol and 31.2 g of (-)-trans-3-(4-methoxyphenyl)glycidic acid methyl ester and the solution is refluxed under nitrogen atmosphere for 2 hours. Diisopropyl ether is added to the reaction mixture, and precipitated crystals are collected by filtration to give 37.6 g of (+)-threo-2-hydroxy-3-(2-amino-5-chlorophenylthio)-3-(4-methoxyphenyl)-propionic acid methyl ester as colorless needles.

Yield: 68 %

m.p. 126 to 129.5 °C

$[\alpha]_D^{20}$ + 248.8 ° (c = 0.3, methanol)

(2) A mixture of 9.5 g of the present product, 0.49 g of p-toluenesulfonic acid monohydrate and 95 ml of xylene is refluxed for 2 hours. After cooling, precipitated crystals are collected by filtration to give 7.5 g of (+)-cis-2-(4-methoxyphenyl)-3-hydroxy-8-chloro-2,3-dihydro-1,5-benzothiazepin-4(5H)-one.

Yield: 86.8 %

m.p. 244 to 245 °C

$[\alpha]_D^{20}$ + 91.6 ° (c = 1.0, dimethylformamide)

Example 2

A mixture of 17.0 g of (+)-threo-2-hydroxy-3-(2-amino-5-chlorophenylthio)-3-(4-methoxyphenyl)-propionic acid, 0.91 g of p-toluenesulfonic acid monohydrate and 220 ml of xylene is refluxed for 2 hours under continuous removal of water. After cooling, precipitated crystals are collected by filtration and washed with ethanol to give 15 g of (+)-cis-2-(4-methoxyphenyl)-3-hydroxy-8-chloro-2,3-dihydro-1,5-benzothiazepin-4(5H)-one.

Yield: 93 %

m.p. 244 to 245 °C

Incidentally, according to the method in which the above reaction is practiced in the absence of p-toluenesulfonic acid in the same manner as described in Japanese Provisional Patent Publication No. 225174/1984, yield of the desired compound is 73 % even after refluxing for 20 hours.

Example 3

A mixture of 17.0 g of (+)-threo-2-hydroxy-3-(2-amino-5-chlorophenylthio)-3-(4-methoxyphenyl)-propionic acid, 0.91 g of p-toluenesulfonic acid monohydrate and 220 ml of toluene is refluxed for 8 hours under continuous removal of water. After cooling, precipitated crystals are collected by filtration and washed with ethanol to give 14.8 g of (+)-cis-2-(4-methoxyphenyl)-3-hydroxy-8-chloro-2,3-dihydro-1,5-benzothiazepin-4(5H)-one.

Yield: 92 %

m.p. 244 to 245 °C

Example 4

(1) In 600 ml of xylene are dissolved 132 g of 2-amino-5-methylthiophenol and 200 g of (±)-trans-3-(4-methylphenyl)glycidic acid methyl ester and the solution is refluxed at 120 to 130 °C under nitrogen atmosphere for 4 hours. After cooling to 40 °C, n-hexane is added to the reaction mixture, and the reaction mixture is stirred and then cooled to 10 °C. Precipitated crystals are collected by filtration to give 218 g of (±)-threo-2-hydroxy-3-(2-amino-5-methylphenylthio)-3-(4-methylphenyl)propionic acid methyl ester as colorless crystals.

Yield: 69 %
m.p. 114 to 116 °C

(2) In 500 g of xylene are dissolved 50 g of the product, 0.57 g of p-toluenesulfonic acid monohydrate is added to the solution and the solution is refluxed for 4 hours. After cooling, precipitated crystals are collected by filtration, washed with xylene to give 39.0 g of (±)-cis-2-(4-methylphenyl)-3-hydroxy-8-methyl-2,3-dihydro-1,5-benzothiazepin-4(5H)-one as colorless crystals.

Yield: 86 %
m.p. 185 to 186 °C

Example 5

In a solution of 200 ml of water and 100 g of methanol are suspended 40 g of (±)-threo-2-hydroxy-3-(2-amino-5-methylphenylthio)-3-(4-methylphenyl)propionic acid methyl ester, 7.4 g of potassium hydroxide are added to the suspension and the mixture is stirred for 30 minutes at 50 to 55 °C. Then, 13.8 g of 35 % hydrochloric acid are added dropwise, and 200 ml of water are added thereto, and the mixture is cooled to 10 °C. Precipitated crystals are collected by filtration and washed with water to give 80 g of (±)-threo-2-hydroxy-3-(2-amino-5-methylphenylthio)-3-(4-methyl phenyl)propionic acid (wet material).

This product (80 g) is suspended in 400 ml of toluene, and 0.4 g of p-toluenesulfonic acid monohydrate is added thereto and the mixture is refluxed for 5 hours under continuous removal of water. After cooling, precipitated crystals are collected by filtration, washed with toluene and dried to give 32.5 g of (±)-cis-2-(4-methylphenyl)-3-hydroxy-8-methyl-2,3-dihydro-1,5-benzothiazepin-4(5H)-one.

Yield: 90 %
m.p. 185 to 186 °C

Example 6

In 400 ml of toluene are dissolved 24.0 g of 2-amino-5-chlorothiophenol and 31.2 g of (-)-trans-3-(4-methoxyphenyl)glycidic acid methyl ester, and the solution is refluxed under nitrogen atmosphere for 2 hours. To the reaction mixture are added 1.43 g of p-toluenesulfonic acid hydrate and 350 ml of xylene and the mixture is refluxed for 2 hours and simultaneously 350 ml of the solvent is distillated. After cooling, precipitated crystals are collected by filtration to give 19.8 g of (+)-cis-2-(4-methoxyphenyl)-3-hydroxy-8-chloro-2,3-dihydro-1,5-benzothiazepin-4(5H)-one.

Physical and chemical properties of the product were identical to those of the product obtained in Example 1.

Example 7

(1) In 420 ml of xylene are dissolved 42 g of 2-amino-5-methylthiophenol and 58 g of (+)-trans-3-(4-methylphenyl)glycidic acid methyl ester, and the solution is refluxed under nitrogen atmosphere for 2 hours. After cooling, n-hexane is added to the reaction mixture and precipitated crystals are collected by filtration to give 65 g of (-)-threo-2-hydroxy-3-(2-amino-5-methylphenylthio)-3-(4-methylphenyl)-propionic acid methyl ester as colorless needles.

Yield: 65 %
m.p. 107 to 109 °C
$[\alpha]_D^{20}$ - 235.4 ° (c = 1, methanol)

(2) A mixture of 20 g of the above product, 0.4 g of p-toluenesulfonic acid monohydrate and 160 ml of xylene is refluxed for 5 hours. After cooling, precipitated crystals are collected by filtration to give 15.7 g of (-)-cis-2-(4-methylphenyl)-3-hydroxy-8-methyl-2,3-dihydro-1,5-benzothiazepin-4(5H)-one.

Yield: 87 %
m.p. 207 to 212 °C
$[\alpha]_D^{20}$ - 120 ° (c = 0.3, methanol)

## Claims

1. A process for preparing 1,5-benzothiazepine derivatives represented by the formula:

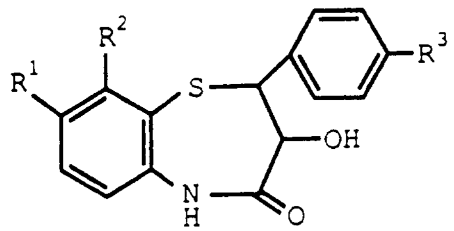

(I)

wherein one of $R^1$ and $R^2$ is a $C_1$ - $C_4$ alkyl group or halogen atom, and the other is hydrogen atom, $R^3$ is a $C_1$ - $C_4$ alkyl group or a $C_1$ - $C_4$ alkoxy group,
which comprises subjecting a propionic acid compound represented by the formula (II):

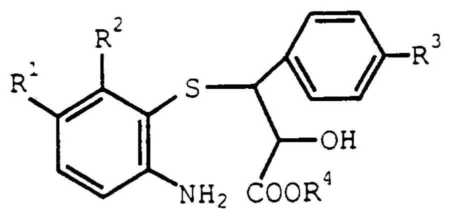

(II)

wherein $R^1$, $R^2$ and $R^3$ have the same meanings as defined above, and $R^4$ represents an ester residue, provided that (1R,2S)-2-phenylcyclohexyl group is disclaimed from said ester residue,
to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, proply group and butyl group, with the proviso that the specific combination of one of $R^1$ and $R^2$ is chlorine atom and the other is hydrogen atom, $R^3$ is methoxy group and $R^4$ is methyl group, and the solvent being chlorinated with a boiling point over 70 ° C and $R^5$ is methyl group or p-tolyl group is disclaimed.

2. A process for preparing 1,5-benzothiazepine derivatives according to Claim 1, wherein the sulfonic acid compound (III) is methanesulfonic acid or p-toluenesulfonic acid.

3. A process for preparing 1,5-benzothiazepine derivatives according to Claim 1, wherein the reaction is carried out in an appropriate solvent under reflux.

4. A process for preparing 1,5-benzothiazepine derivatives according to Claim 1, wherein the sulfonic acid (III) is used at an amount of 0.5 to 10 w/w % based on the compound (II).

5. A process for preparing 1,5-benzothiazepine derivatives according to Claim 4, wherein the sulfonic acid (III) is used at an amount of 1 to 6 w/w % based on the propionic acid compound (II).

**6.** A process for preparing optically active cis-1,5-benzothiazepine derivatives represented by the formula:

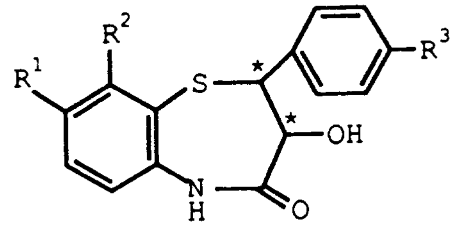

(I)

wherein one of $R^1$ and $R^2$ is a $C_1$ - $C_4$ alkyl group or halogen atom, and the other is hydrogen atom, $R^3$ is a $C_1$ - $C_4$ alkyl group or a $C_1$ - $C_4$ alkoxy group, and * represents an asymmetric carbon atom,
which comprises reacting a thiophenol compound represented by the formula (IV):

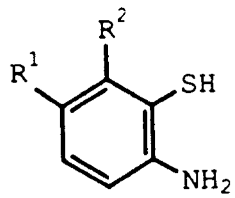

(IV)

wherein $R^1$ and $R^2$ have the same meanings as defined above,
with an optically active trans-glycidic acid ester represented by the formula:

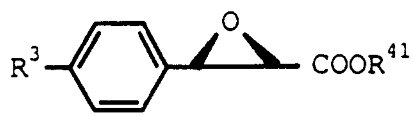

(V)

wherein $R^3$ has the same meaning as defined above, and $R^{41}$ represents an ester residue, provided that (1R,2S)-2-phenylcyclohexyl group is disclaimed from said ester residue,
to form an optically active threo-propionic acid ester compound represented by the formula:

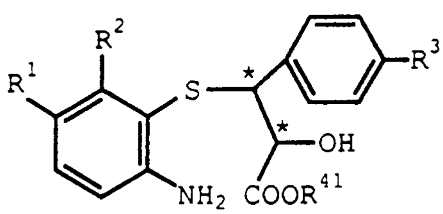

(II - a)

wherein $R^1$, $R^2$, $R^3$, $R^{41}$ and * have the same meanings as defined above,
and then subjecting the above compound (II - a) to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, proply group and butyl group, with the proviso that the specific combination of one of $R^1$ and $R^2$ is chlorine atom and the other is hydrogen atom, $R^3$ is methoxy group and $R^4$ is methyl group, and the solvent being chlorinated with a boiling point over 70 °C and $R^5$ is methyl group or p-tolyl group is disclaimed.

**7.** A process for preparing 1,5-benzothiazepine derivatives according to Claim 6, wherein the reaction of the thiophenol compound (IV) and the optically active trans-glycidic acid ester (V) is carried out in an appropriate solvent under heating.

**8.** A process for preparing 1,5-benzothiazepine derivatives according to Claim 6, wherein the sulfonic acid compound (III) is methanesulfonic acid or p-toluenesulfonic acid.

**9.** A process for preparing 1,5-benzothiazepine derivatives according to Claim 6, wherein the intramolecular ring closing reaction is carried out in an appropriate solvent under reflux.

**10.** A process for preparing 1,5-benzothiazepine derivatives according to Claim 6, wherein the sulfonic acid (III) is used at an amount of 0.5 to 10 w/w % based on the compound (II - a).

**11.** A process for preparing 1,5-benzothiazepine derivatives according to Claim 10, wherein the sulfonic acid (III) is used at an amount of 1 to 6 w/w % based on the propionic acid compound (II - a).

**12.** A process for preparing 3-acetoxy-5-(β-dimethylaminoethyl)-1,5-benzothiazepine derivatives represented by the formula:

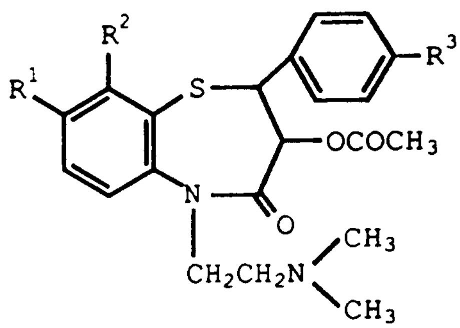

(VI)

wherein one of $R^1$ and $R^2$ is a $C_1$ - $C_4$ alkyl group or halogen atom, and the other is hydrogen atom, $R^3$ is a $C_1$ - $C_4$ alkyl group or a $C_1$ - $C_4$ alkoxy group,
or a pharmaceutically acceptable salt thereof which process comprises: subjecting a propionic acid compound represented by the formula (II):

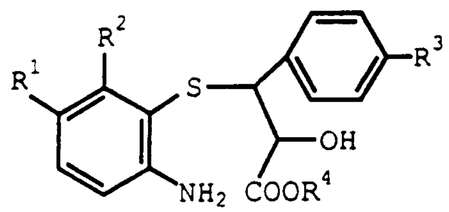

(II)

wherein $R^1$, $R^2$ and $R^3$ have the same meanings as defined above, and $R^4$ represents an ester residue, provided that (1R,2S)-2-phenylcyclohexyl group is disclaimed from said ester residue,
to intramolecular ring closing reaction in the presence of a sulfonic acid compound represented by the formula (III):

$R^5SO_3H$ (III)

wherein $R^5$ represents methyl group, ethyl group, propyl group, butyl group or phenyl group which may be substituted by at least one selected from methyl group, ethyl group, propyl group and butyl group,
to give a compound represented by the formula (I):

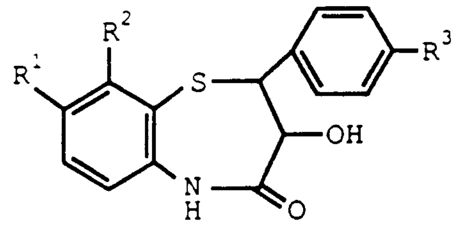

(I)

wherein $R^1$, $R^2$ and $R^3$ have the same meanings as defined above,
and converting the compound (I) to the corresponding 3-acetoxy-5-(β-dimethylaminoethyl)-1,5-benzothiazepine derivative or a pharmaceutically acceptable salt thereof in a known method, with the proviso that the specific combination of one of $R^1$ and $R^2$ is chlorine atom and the other is hydrogen atom, $R^3$ is methoxy group and $R^4$ is methyl group, and the solvent being chlorinated with a boiling point over 70 ° C and $R^5$ is methyl group or p-tolyl group is disclaimed.

**Patentansprüche**

1. Verfahren zur Herstellung von 1,5-Benzothiazepinderivaten, welche durch die Formel

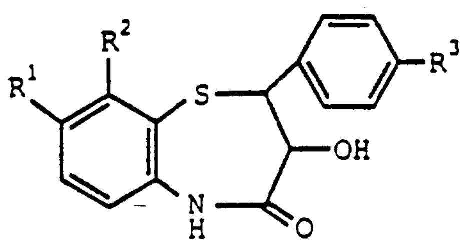

(I)

dargestellt werden, worin eines von $R^1$ und $R^2$ eine $C_1$-$C_4$-Alkylgruppe oder ein Halogenatom ist und das andere ein Wasserstoffatom ist, $R^3$ eine $C_1$-$C_4$-Alkylgruppe oder eine $C_1$-$C_4$-Alkoxygruppe ist, umfassend das Aussetzen einer Propionsäureverbindung, welche durch die Formel (II):

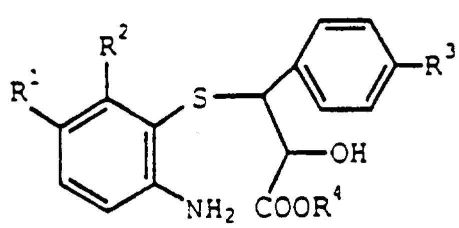

(II)

dargestellt wird, worin $R^1$, $R^2$ und $R^3$ dieselben Bedeutungen wie vorstehend definiert haben und $R^4$ einen Esterrest bedeutet, mit der Maßgabe, daß die (1R, 2S)-2-Phenylcyclohexylgruppe aus dem Esterrest ausgeschlossen ist,
einer intramolekularen Ringschlußreaktion in der Gegenwart einer Sulfonsäureverbindung, welche durch die Formel (III):

$R^5SO_3H$ (III)

dargestellt wird, worin $R^5$ eine Methylgruppe, Ethylgruppe, Propylgruppe, Butylgruppe oder Phenylgruppe, die durch mindestens eine, aus der Methylgruppe, Ethylgruppe, Propylgruppe und Butylgruppe, substituiert sein kann, bedeutet, mit der Maßgabe, daß die spezifische Kombination, daß $R^1$ oder $R^2$ ein Chloratom und das jeweils andere ein Wasserstoffatom ist, $R^3$ eine Methoxygruppe ist und $R^4$ eine

Methylgruppe ist und das Losungsmittel chloriert ist, mit einem Siedepunkt über 70 °C und $R^5$ eine Methylgruppe oder p-Tolylgruppe ist, ausgeschlossen ist.

2. Verfahren zum Herstellen von 1,5-Benzothiazepinderivaten nach Anspruch 1, worin die Sulfonsäureverbindung (III) Methansulfonsäure oder p-Toluolsulfonsäure ist.

3. Verfahren zum Herstellen von 1,5-Benzothiazepinderivaten nach Anspruch 1, worin die Reaktion in einem geeigneten Lösungsmittel unter Rückfluß durchgeführt wird.

4. Verfahren zum Herstellen von 1,5-Benzothiazepinderivaten nach Anspruch 1, worin die Sulfonsäure (III) in einer Menge von 0,5 bis 10 Gew/Gew.-%, basierend auf der Verbindung (II) verwendet wird.

5. Verfahren zum Herstellen von 1,5-Benzothiazepinderivaten nach Anspruch 4, worin die Sulfonsäure (III) in einer Menge von 1 bis 6 Gew/Gew.-%, basierend auf der Propionsäureverbindung (II) verwendet wird.

6. Verfahren zum Herstellen optisch aktiver cis-1,5-Benzothiazepinderivate, welche durch die Formel:

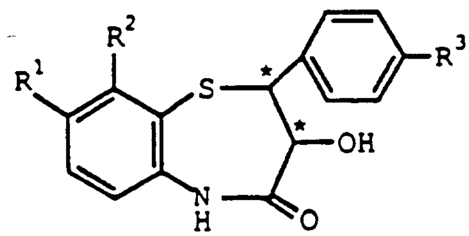

(I)

dargestellt werden, worin eines von $R^1$ und $R^2$ eine $C_1$-$C_4$-Alkylgruppe oder ein Halogenatom ist und das andere ein Wasserstoffatom ist, $R^3$ eine $C_1$-$C_4$-Alkylgruppe oder eine $C_1$-$C_4$-Alkoxygruppe ist und worin * ein asymmetrisches Kohlenstoff bedeutet,
umfassend das Umsetzen einer Thiophenolverbindung, welche durch die Formel (IV):

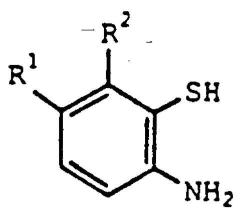

(IV)

dargestellt wird, worin $R^1$ und $R^2$ dieselben Bedeutungen wie vorstehend definiert haben, mit einem optisch aktiven trans-Glycidsäureester, welcher durch die Formel:

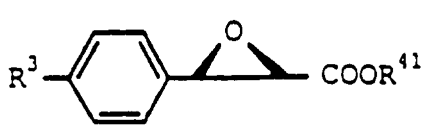

(V)

dargestellt wird, worin $R^3$ dieselbe Bedeutung wie vorstehend definiert hat und $R^{41}$ einen Esterrest bedeutet, mit der Maßgabe, daß die (1R,2S)-2-Phenylcyclohexylgruppe von dem Esterrest ausgeschlossen ist,
um eine optisch aktive threo-Propionsäureesterverbindung herzustellen, welche durch die Formel:

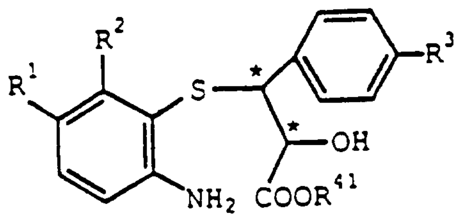

(II - a)

dargestellt wird, worin $R^1$, $R^2$, $R^3$, $R^{41}$ und * dieselben Bedeutungen wie vorstehend definiert haben, und dann Aussetzen der vorstehenden Verbindung (II-a) einer intramolekularen Ringschlußreaktion in der Gegenwart einer Sulfonsäureverbindung, welche durch die Formel (III):

$R^5SO_3H$ (III)

dargestellt wird, worin $R^5$ eine Methylgruppe, Ethylgruppe, Propylgruppe, Butylgruppe oder Phenylgruppe, die durch mindestens eine, ausgewählt aus der Methylgruppe, Ethylgruppe, Propylgruppe und Butylgruppe, substituiert sein kann, darstellt, mit der Maßgabe, daß die spezifische Kombination, daß $R^1$ oder $R^2$ ein Chloratom und das jeweils andere ein Wasserstoffatom ist, $R^3$ eine Methoxygruppe ist und $R^4$ eine Methylgruppe ist und das Lösungsmittel chloriert ist, mit einem Siedepunkt über 70 °C und $R^5$ eine Methylgruppe oder p-Tolylgruppe ist, ausgeschlossen ist.

**7.** Verfahren zum Herstellen von 1,5-Benzothiazepinderivaten nach Anspruch 6, worin die Reaktion der Thiophenolverbindung (IV) und des optisch aktiven trans-Glycidsäureesters (V) in einem geeigneten Lösungsmittel unter Erhitzen ausgeführt wird.

**8.** Verfahren zum Herstellen von 1,5-Benzothiazepinderivaten nach Anspruch 6, worin die Sulfonsäureverbindung (III) Methansulfonsäure oder p-Toluolsulfonsäure ist.

**9.** Verfahren zum Herstellen von 1,5-Benzothiazepinderivaten nach Anspruch 6, worin die intramolekulare Ringschlußreaktion in einem geeigneten Lösungsmittel unter Rückfluß ausgeführt wird.

**10.** Verfahren zum Herstelle von 1,5-Benzothiazepinderivaten nach Anspruch 6, worin die Sulfonsäure (III) in einer Menge von 0,5 bis 10 Gew/Gew.-%, basierend auf der Verbindung (II-a) verwendet wird.

**11.** Verfahren zum Herstellen von 1,5-Benzothiazepinderivaten nach Anspruch 10, worin die Sulfonsäure (I) in einer Menge von 1 bis 6 Gew/Gew.-%, basierend auf der Propionsäureverbindung (II-a) verwendet wird.

**12.** Verfahren zum Herstellen von 3-Acetoxy-5-(β-dimethylaminoethyl)-1,5-benzothiazepinderivaten, welche durch die Formel:

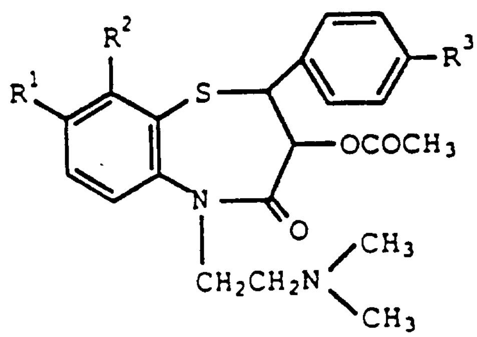

(VI)

dargestellt sind, worin eines von $R^1$ und $R^2$ eine $C_1$-$C_4$-Alkylgruppe oder ein Halogenatom ist und das

andere ein Wasserstoffatom ist, $R^3$ eine $C_1$-$C_4$-Alkylgruppe oder eine $C_1$-$C_4$-Alkoxygruppe ist, oder einem pharmazeutisch verträglichen Salz davon, wobei das Verfahren umfaßt: Aussetzen einer Propionsäureverbindung, welche durch die Formel (II):

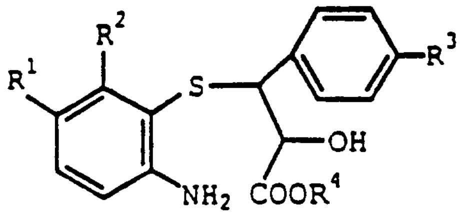

(II)

dargestellt ist, worin $R^1$, $R^2$ und $R^3$ die gleichen Bedeutungen haben, wie vorstehend definiert und $R^4$ einen Esterrest bedeutet, mit der Maßgabe, daß die (1R,2S)-2-Phenylcyclohexylgruppe aus dem Esterrest ausgeschlossen ist, einer intramolekularen Ringschlußreaktion in der Gegenwart einer Sulfonsäureverbindung, welche durch die Formel (III):

$R^5SO_3H$ (III)

dargestellt ist, worin $R^5$ eine Methylgruppe, Ethylgruppe, Propylgruppe, Butylgruppe oder Phenylgruppe, die durch mindestens eine, ausgewählt aus der Methylgruppe, Ethylgruppe, Propylgruppe oder Butylgruppe substituiert sein kann, bedeutet,
um eine Verbindung zu ergeben, welche durch die Formel (I):

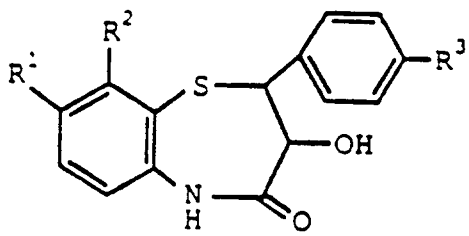

(I)

dargestellt ist, worin $R^1$, $R^2$ und $R^3$ die gleichen Bedeutungen wie vorstehend definiert haben, und Überführen der Verbindung (I) in das entsprechenden 3-Acetoxy-5-($\beta$-dimethylaminoethyl)-1,5-benzothiazepinderivat oder einem pharmazeutisch verträglichen Salz davon durch ein bekanntes Verfahren, mit der Maßgabe, daß die spezifische Kombination, daß $R^1$ oder $R^2$ ein Chloratom und das jeweils andere ein Wasserstoffatom ist, $R^3$ eine Methoxygruppe ist und $R^4$ eine Methylgruppe ist und das Lösungsmittel chloriert ist, mit einem Siedepunkt über 70 °C und $R^5$ eine Methylgruppe oder p-Tolylgruppe ist, ausgeschlossen ist.

## Revendications

1. Procédé de préparation de dérivés de la 1,5-benzothiazépine représentés par la formule :

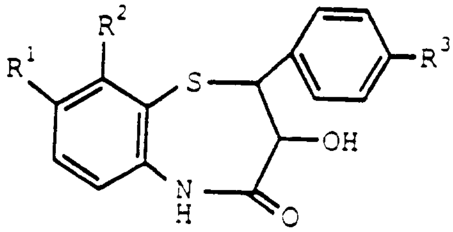

(I)

dans laquelle l'un des symboles $R^1$ et $R^2$ représente un radical alkyle en $C_1$-$C_4$ ou un atome d'halogène, cependant que l'autre de ces symboles représente un atome d'hydrogène, $R^3$ représente un radical alkyle en $C_1$-$C_4$, ou un radical alcoxy en $C_1$-$C_4$,
caractérisé en ce que l'on soumet un composé de l'acide propionique représenté par la formule (II) :

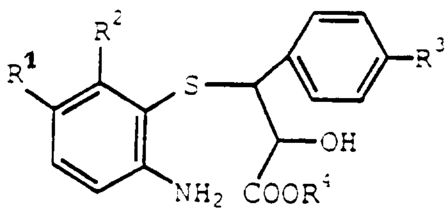

(II)

dans laquelle $R^1$, $R^2$ et $R^3$ possèdent les mêmes significations que celles qui leur ont été attribuées ci-dessus et $R^4$ représente un reste d'ester, avec la condition que le radical (1R,2S)-2-phénylcyclohexyle soit exclu du reste d'ester précité,
à une réaction de fermeture du cycle intramoléculaire en présence d'un composé de l'acide sulfonique représenté par la formule (III) :

$R^5SO_3H$ (III)

dans laquelle $R^5$ représente le radical méthyle, le radical éthyle, le radical propyle, le radical butyle, ou le radical phényle, qui peut être substitué par au moins un des substituants choisis dans le groupe formé par le radical méthyle, le radical éthyle, le radical propyle et le radical butyle, avec le préalable que soit exclue la combinaison spécifique conformément à laquelle l'un des symboles $R^1$ et $R^2$ représente un atome de chlore cependant que l'autre représente un atome d'hydrogène, $R^3$ représente le radical méthoxy et $R^4$ représente le radical méthyle, le solvant est chloré et possède un point d'ébullition supérieur à 70 °C et $R^5$ représente le radical méthyle ou le radical p-tolyle.

2. Procédé de préparation de dérivés de la 1,5-benzothiazépine suivant la revendication 1, caractérisé en ce que le composé de l'acide sulfonique (III) est l'acide méthanesulfonique, ou l'acide p-toluènesulfonique.

3. Procédé de préparation de dérivés de la 1,5-benzothiazépine suivant la revendication 1, caractérisé en ce que l'on entreprend la réaction dans un solvant approprié sous reflux.

4. Procédé de préparation de dérivés de la 1,5-benzothiazépine suivant la revendication 1, caractérisé en ce que l'on utilise l'acide sulfonique (III) dans la proportion de 0,5 à 10% p/p sur base du composé (II).

5. Procédé de préparation de dérivés de la 1,5-benzothiazépine suivant la revendication 4, caractérisé en ce que l'on utilise l'acide sulfonique (III) en une proportion de 1 à 6% p/p sur base du composé de l'acide propionique (II).

6. Procédé de préparation de dérivés de la cis-1,5-benzothiazépine optiquement actifs représentés par la formule :

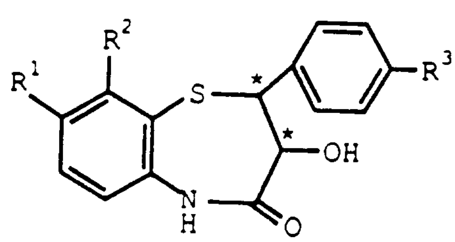

(I)

dans laquelle l'un des symboles $R^1$ et $R^2$ représente un radical alkyle en $C_1$-$C_4$, ou un atome d'halogène et l'autre de ces symboles représente un atome d'hydrogène, $R^3$ représente un radical alkyle en $C_1$-$C_4$, ou un radical alcoxy en $C_1$-$C_4$ et * représente un atome de carbone asymétrique, caractérisé en ce que l'on fait réagir un composé du thiophénol représenté par la formule IV :

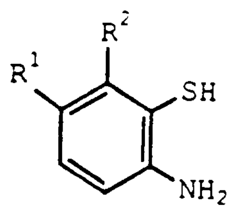

(IV)

dans laquelle $R^1$ et $R^2$ possèdent les mêmes significations que celles qui leur ont été attribuées ci-dessus, avec un ester de l'acide trans-glycidique optiquement actif représenté par la formule :

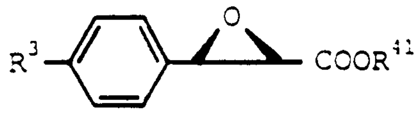

(V)

dans laquelle $R^3$ possède les mêmes significations que celles qui lui ont été attribuées ci-dessus et $R^{41}$ représente un reste d'ester, avec la condition que le radical (1R, 2S)-2-phénylcyclohexyle soit exclu dudit reste d'ester,
pour former un composé d'ester de l'acide thréo-propionique optiquement actif représenté par la formule :

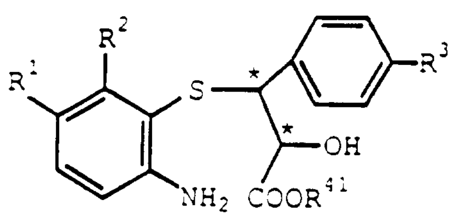

(II - a)

dans laquelle $R^1$, $R^2$, $R^3$, $R^{41}$ et * possèdent les mêmes significations que celles qui leur ont été précédemment attribuées
et on soumet ensuite le composé susmentionné (II-a) à une réaction de fermeture de cycle intramoléculaire en présence d'un composé de l'acide sulfonique représenté par la formule (III) :

$R^5SO_3H$ (III)

dans laquelle $R^5$ représente le radical méthyle, le radical éthyle, le radical propyle, le radical butyle, le radical phényle, qui peut être substitué par au moins un substituant choisi parmi le radical méthyle, le radical éthyle, le radical propyle et le radical butyle, avec le préalable que soit exclue la combinaison spécifique conformément à laquelle l'un des symboles $R^1$ et $R^2$ représente un atome de chlore cependant que l'autre représente un atome d'hydrogène, $R^3$ représente le radical méthoxy et $R^4$ représente le radical méthyle, le solvant est chloré et possède un point d'ébullition supérieur à 70 °C et $R^5$ représente le radical méthyle ou le radical p-tolyle.

**7.** Procédé de préparation de dérivés de la 1,5-benzothiazépine suivant la revendication 6, caractérisé en ce que l'on réalise la réaction du composé de thiophénol (IV) avec l'ester de l'acide trans-glycidique optiquement actif (V) dans un solvant approprié et sous chauffage.

**8.** Procédé de préparation de dérivés de la 1,5-benzothiazépine suivant la revendication 6, caractérisé en ce que le composé de l'acide sulfonique (III) est l'acide méthanesulfonique, ou l'acide p-toluènesulfonique.

**9.** Procédé de préparation de dérivés de la 1,5-benzothiazépine suivant la revendication 6, caractérisé en ce que la réaction de fermeture du cycle intramoléculaire se réalise dans un solvant approprié, sous reflux.

**10.** Procédé de préparation de dérivés de la 1,5-benzothiazépine suivant la revendication 6, caractérisé en ce que l'on utilise l'acide sulfonique (III) en une proportion de 0,5 à 10% p/p sur base du composé (II-a).

**11.** Procédé de préparation de dérivés de la 1,5-benzothiazépine suivant la revendication 10, caractérisé en ce que l'on utilise l'acide sulfonique (III) en une proportion de 1 à 6% p/p sur base du composé de l'acide propionique (II-a).

**12.** Procédé de préparation de dérivés de la 3-acétoxy-5-($\beta$-diméthylaminoéthyl)-1,5-benzothiazépine représentés par la formule :

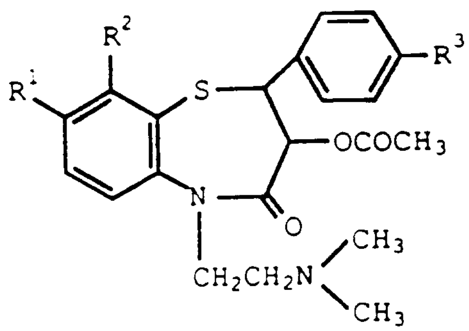

(VI)

dans laquelle l'un des symboles $R^1$ et $R^2$ représente un radical alkyle en $C_1$-$C_4$ ou un atome d'halogène cependant que l'autre représente un atome d'hydrogène, $R^3$ représente un radical alkyle en $C_1$-$C_4$, ou un radical alcoxy en $C_1$-$C_4$,
ou d'un sel pharmaceutiquement acceptable de ces dérivés, caractérisé en ce que l'on soumet un composé de l'acide propionique représenté par la formule (II) :

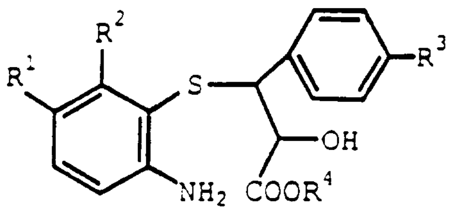

(II)

dans laquelle $R^1$, $R^2$ et $R^3$ possèdent les mêmes significations que celles qui leur ont été précédemment attribuées et $R^4$ représente un reste d'ester, avec la condition que le radical (1R, 2S)-2-phénylcyclohexyle soit exclu du dudit reste d'ester,
à une réaction de fermeture du cycle intramoléculaire en présence d'un composé de l'acide sulfonique représenté par la formule (III) :

$R^5SO_3H$ (III)

dans laquelle $R^5$ représente le radical méthyle, le radical éthyle, le radical propyle, le radical

butyle, ou le radical phényle, qui peut être substitué par au moins l'un des substituants choisis parmi le radical méthyle, le radical éthyle, le radical propyle et le radical butyle,
de manière à obtenir un composé représenté par la formule (I) :

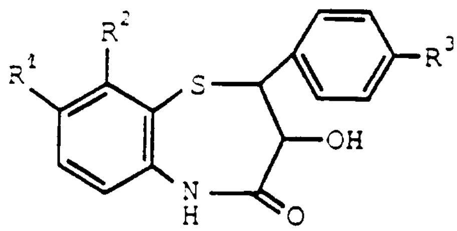

(I)

dans laquelle $R^1$, $R^2$ et $R^3$ possèdent les mêmes significations que celles qui leur ont été précédemment attribuées,
et on convertit le composé (I) en le dérivé de la 3-acétoxy-5-($\beta$-diméthylaminoéthyl)-1,5-benzothiazépine correspondant, ou un sel pharmaceutiquement acceptable de ce dérivé, selon une méthode connue, avec le préalable que soit exclue la combinaison spécifique conformément à laquelle l'un des symboles $R^1$ et $R^2$ représente un atome de chlore cependant que l'autre représente un atome d'hydrogène, $R^3$ représente le radical méthoxy et $R^4$ représente le radical méthyle, le solvant est chloré et possède un point d'ébullition supérieur à 70 °C et $R^5$ représente le radical méthyle ou le radical p-tolyle.